# EUROPEAN PATENT SPECIFICATION

(11) **EP 3 999 127 B1**
(45) Date of publication and mention of the grant of the patent: **04.03.2026**
(21) Application number: 21715878.1
(22) Date of filing: 29.03.2021
(51) Int. Cl.: A61L 9/015, A61L 9/04, A61L 9/14, A61L 9/18, A61L 2/025, A61L 2/03, A61L 2/08, A61L 2/10, A61L 9/20, A61L 9/12

(54) **FLUID TREATMENT UNIT**
FLÜSSIGKEITSBEHANDLUNGSEINHEIT
DISPOSITIF DE TRAITEMENT DE FLUIDE

(30) Priority: 09.04.2020 ES 202030294
(43) Date of publication of application: 25.05.2022
(73) Proprietor: Monagas Asensio, Pedro, 08211 Castellar Del Valles (Barcelona) (ES); Oltra Orta, Jordi, 08208 Sabadell Barcelona (ES)
(72) Inventor: MONAGAS ASENSIO, Pedro, 08211 Castellar Del Valles (Barcelona) (ES); OLTRA ORTA, Jordi, 08208 Sabadell Barcelona (ES)
(74) Representative: ZBM Patents - Zea, Barlocci & Markvardsen
(86) International application number: PCT/EP2021/058100
(87) International publication number: WO 2021/204573

(56) References cited:
- EP-A2- 1 891 980
- WO-A1-2006/111088
- WO-A1-2010/093796
- WO-A1-2016/094658
- CN-A- 104 707 477
- CN-A- 108 079 330
- CN-U- 206 786 911
- JP-A- 2002 085 978
- KR-A- 20190 067 633
- KR-B1- 101 708 799
- US-A1- 2004 251 122
- US-A1- 2006 104 858
- US-A1- 2007 221 582
- US-A1- 2016 136 624
- US-A1- 2018 297 865

## Description

### TECHNICAL FIELD

This invention refers to a fluid treatment unit such as a ventilation device with purification, germicidal and electro-photocatalytic functions. It is applicable to conditioning systems that treat fluids in liquid or gaseous form (air conditioning, air curtains, etc.) and to evacuation and treatment of fluids such as fumes in kitchens and industrial facilities.

It has applications in the fields of fluid treatment, ventilation and air conditioning.

### STATE OF THE ART

Ventilation devices providing photocatalytic installations, purifiers, etc. are known in the state of the art. FR2785370 or CN107305052 are examples of such systems.

These devices enable good control and catalysis of ventilated gases, but have limited germicidal action.

The use of titanium dioxide coatings for water purification, as in US2020070135, is also known.

Useful prior art documents are US 2007/221582 A1, WO 2016/094658 A1, US 2004/251122 A1, KR 2019 0067633 A, JP 2002 085978 A and WO 2006/111088 A1.

The applicant does not know of a fluid treatment device that enables similar advantages to be obtained or that can be considered similar to the invention.

### BRIEF EXPLANATION OF THE INVENTION

The invention consists of a fluid treatment device that can be used, for example, in ventilation in accordance with the claims. Its various embodiments solve problems in the state of the art and provide notable advantages.

A fluid treatment device according to claim 1 of the present invention is provided.

The device is based on the germicidal effect of materials with electro-photocatalysis properties that emit radiation at the ultraviolet frequency. Titanium dioxide, metal nitrides (such as titanium, chromium, etc.), strontium titanate and other metal oxides, for example, with the ability to absorb radiation in the visible spectrum, modified with transition metal dimers and trimers (for example using glycine, diphenylamine or p-amino benzoic acid), especially when illuminated with UV light. In this case, the desirable effect is intensified by the addition of an electrocatalytic effect which converts illumination with UV light into an accessory.

The device proposed in the invention may comprise elements capable of transferring °O, °H and °OH radicals produced by the various reactions to treat the gas. The maximum generation of radicals is controlled by the technology explained herein with the aim of achieving a condition of cleanliness and comfort by means of the effect of the aforesaid radicals in purification and disinfection of the air.

This reactive compound generator with oxidizing action creates a barrier to maintain a constant, invisible flow of air that contains °OH hydroxyl radicals. It generates a clean, disinfected atmosphere free of environmental pollutants from both inside and outside the treated space, in other words serves as a barrier, "oxides" and eliminates viruses, bacteria, dust, suspended particles, organic pollutants in suspension, odours, insects and that blocks both hot and cold air currents.

On the basis of Advanced Oxidation Processes (AOP) technology, the scope of the invention enables the reduction of proliferation, up to disappearance, of volatile organic compounds, pollutants, solid particles in suspension, fungi, spores, moulds, bacteria, and viruses to zero and also eradicates or mitigates bad odours, fumes, mites, etc.

The treatment device is provided with an intake for the feed fluid, the fluid being a gas such as the air in a room, an evacuation pipe, an extractor or a wastewater conduit. It is also fitted with a pump or compressor that provides movement of the fluid and an outlet for the treated fluid. This fluid outlet could be an air curtain or other type of air jet in ventilation devices. The device also comprises a semiconductor coating made of materials with electro-photocatalysis properties that emits radiation at the ultraviolet frequency. For example, titanium oxides (preferably titanium dioxide), metal nitrides (preferably aluminium) and other metal oxides with the ability to absorb radiation in the visible spectrum modified with transition metal dimers and trimers. It may be accompanied by an underlying layer or base of titanium, cobalt, copper, silver, zinc or aluminium on a support in the circuit and, optionally, a UV light source that illuminates said coating. The semiconductor coating is earthed and illuminated by UV light or is provided with a polarised direct current power source. To facilitate this electrical contact the preferred method of application of this coating is a physical vapour deposition (PVD) process.

Various reduced titanium oxides are known. For example, Ti₃O₅ is a purple-coloured semiconductor produced by reducing TiO₂ with hydrogen at high temperatures. Other common oxides are titanium (III) oxide Ti₂O₃ and titanium (II) oxide TiO.

Titanium nitride (TiN) is as hard as sapphire and silicon carbide (9.0 on the Mohs hardness scale) and is commonly used as a coating for industrial cutting tools. It is also used as a gold-coloured decorative coating and as a barrier-metal in semiconductor manufacturing.

All the electrons in the atomic structure of these semiconductor materials have a bonding role and are endowed with energies associated with the valence band while the conduction band remains in vacant electronic states. At higher temperatures some valence electrons can absorb enough energy to break free from the bond and move through the semiconductor material as free electrons. In this event, their energy is transferred to the conduction band. The higher the temperature the greater the number of conduction electrons, but even at room temperature this number is high enough to enable us to confirm that the semiconductor conducts electric current.

Once it has been converted into a conduction electron, any valence electron leaves a vacancy so that, if an electric field is applied to the semiconductor, this vacancy can be filled by another valence electron, which in turn leaves a new vacancy in the orbit that it occupied. The effect of these substitutions is that an e+ charge moving in the direction of the electric field (polarity) is created.

Carrier recombination occurs simultaneously with the electron-hole pair thermal generation process. Some electrons in the conduction band can lose energy by emitting it in the form of photons, the wavelength of which is determined by the materials used. This radiation has germicidal properties if its wavelength is roughly 200-300 nm.

Coating of materials with electro-photocatalysis such as titanium dioxide or metal nitride is performed inside the compressor or pump, on the impulsion blades. This ensures that the turbulence mixes the elements and maximises contact of the fluid with the coating.

The coating is connected to a direct current power source in the preferred embodiment for treatment of gases. This is less important in the case of liquids.

When treating gases, the preferred embodiment comprises an ozonation unit and one or more hydrogen peroxide nebulizers. These units will preferably be arranged close together so that the synergy between them produces more free radicals. Furthermore, they should be placed upstream of the titanium dioxide or metal nitride coating to ensure that the effect is concentrated in said area. The turbulence increases the effect of the coating if it is applied in the compressor.

The nebulizer should preferably be comprised of a sonolysis head such as a piezoelectric membrane that vibrates at the desired frequency. This embodiment is based on application of ultrasound waves of approximately 670 kHz to the fluid to generate the physicochemical reaction known as *sonolysis.* In this reaction, the water microparticle (H₂O), in our case containing hydrogen peroxide, is dissociated into highly-reactive oxygen species such as the hydroxyl radical (OH°). This radical facilitates oxidation and degradation of other compounds with lower molecular masses.

Other variants will be mentioned throughout this specification.

### DESCRIPTION OF THE DRAWINGS

The following figures are provided in the interests of a better understanding of the invention.
Figure 1: Simplified diagram of an embodiment of the device applied to ventilation.
Figure 2: Detail of a method of applying the coating.
Figure 3: Simplified diagram of a second coating application method on the compressor blades. It shows a schematic cross-section along the axis of rotation of the rotor.

### MODES OF EMBODIMENT OF THE INVENTION

The following is a brief description of one possible embodiment of the invention, as an illustrative and non-limiting example applied to a ventilation device.

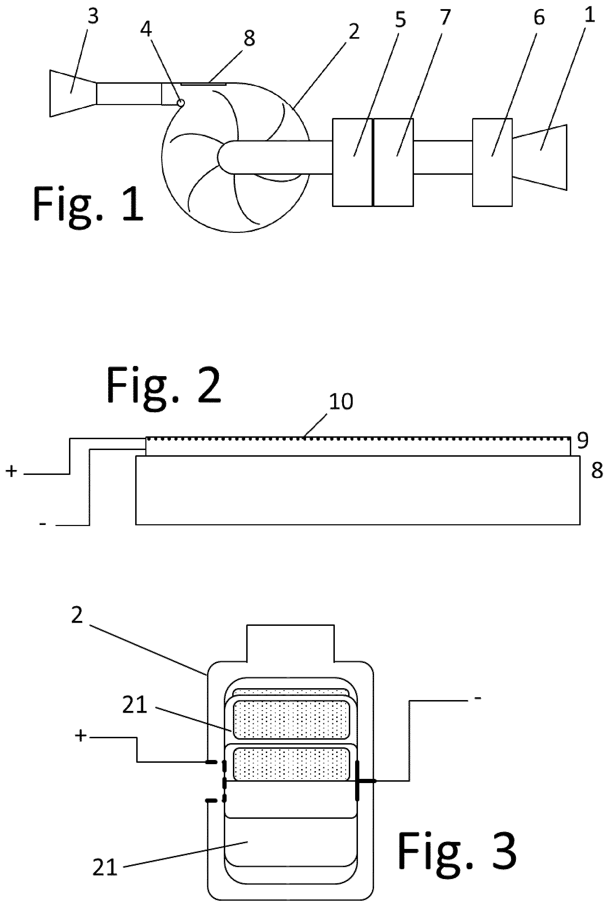

The ventilation and purification device shown in the diagram of Figure 1 is comprised of various components. Firstly, a fluid - generally air - intake (1) from outside or inside the area to be ventilated. It is also fitted with a pump or compressor (2) that enables controlled movement of the gas along the conduits. A fluid outlet (3) expels the gas into the area to be ventilated, a chimney or wherever required depending on the application. For example, the fluid outlet (3) could be an air curtain placed above a portico or door.

The ventilation device has an electro-photocatalyst coating on a support (8) somewhere in its conduits. The coating is made of materials with electro-photocatalysis properties that emit radiation at the ultraviolet frequency. Titanium oxide, a metal nitride, strontium titanate and other metal oxides with the ability to absorb in the visible spectrum, modified with transition metal dimers and trimers, are mentioned as examples. The coating is connected to a conductor. The conductor may form part of a direct-current circuit or be connected to earth, in which case the coating is illuminated by at least one UV light (4) emitted, for example, by one or more LEDs. This connection endows the photocatalyst with germicidal properties. In the first embodiment - with direct current - a UV light source can also be added (4).

The direct current power supply may be achieved by connecting both ends of the coating to the associated conductor and pole of a battery or transformer. Since titanium dioxide or metal nitrides are not conductive, the coating is preferably applied over a conductive layer (9) or base made of titanium, copper, zinc or aluminium, which reinforces their beneficial effects. Other metals or conductors are possible but are not preferred. The photocatalyst may, for example, be composed of a deposition (10) of titanium dioxide or metal nitride on the underlying layer (9) or base by vapour phase deposition (VPD) technology (figure 2). The base or underlying layer (9) will normally be arranged on a support (8) made of metal or ceramics that forms part of the compressor or the piping.

The electro-photocatalyst is located in the compressor (2) where a turbulent regime is generated that maximises contact of the photocatalyst with the gas, and is applied to the blades (21) of the compressor (2). An example of an embodiment is shown in Figure 3, in which the coating is applied to the blades (21) and the rotor is connected on both sides, by means of the shaft, to the two poles.

The ventilation device shown as an example of an air purification unit has other optional features of interest such as an ozonation unit (5), one or more filters (6) and deionized water or other compounds.

The ozonation unit (5) will control the ozone emission to ensure that the safe concentration (0.05 ppm) is not exceeded. It can work by corona discharge and will preferably be placed near a hydrogen peroxide nebulizer (7). This arrangement favours a reaction between hydrogen peroxide and ozone that enhances the presence of the hydroxyl radical, an oxidation agent of unwanted particles and substances. The turbulence fosters increased dispersion if they are also arranged upstream of the compressor (2). The hydroxyl radical creates a cascade effect in which the substances mutually oxidize in accordance with their REDOX potential and have strong bactericidal, antiviral and antifungal properties.

The nebulization (7) of the hydrogen peroxide is preferably produced by sonolysis. To do so, the nebulizer (7) is comprised of a cartridge with a wick that carries the liquid to a head by capillary action. The head is fitted with a transducer with a piezoelectric membrane. Vibration of the piezoelectric membrane achieves three effects: it nebulizes the hydrogen peroxide, dissociates it into hydroxyl and other radicals and generates resonance of the chitin that forms part of the exoskeleton of mites and many fungi and spores and micronizes the water particles to create cold vapour. A wave, preferably square, with a frequency of 670 kHz must be obtained in the oscillator to achieve these effects. In this case, the resonant frequency of chitin is harmonic 17.

### Example:

Two ceramic plates with a coating of TiO₂ are employed. A first plate (A) not modified in any way, a second plate (B) connected to earth by means of a copper wire and a third plate (C) with titanium sintering, TiO₂ coating and also earthed. A 4-ml sample of *E. Coli* solution at 10⁸ CFU/ml is placed on each plate. They are illuminated with stable, 254-nm wavelength UV light at 25 °C for 3 hours. Samples are taken at 15, 30, 45 and 60 minutes.

The samples are analysed and the percentage of bacteria eliminated at each moment is calculated.

| **Plate** | **15 min** | **30 min** | **45 min** | **60 min** |
|---|---|---|---|---|
| **A** | 30.05% | 43.01% | 37.72% | 49.59% |
| **B** | 32.60% | 42.65% | 42.28% | 66.21% |
| **C** | 27.12% | 32.42% | 63.47% | 76.07% |

## Claims

1. A fluid treatment device with a fluid intake (1), a pump or compressor (2) and a fluid outlet (3), the device comprising a support (8) with an internal area with a coating of materials with electro-photocatalysis properties that emits radiation in the ultraviolet frequency modified with transition metal dimers and trimers, the support (8) being either:
connected to earth and illuminated by UV light (4) or
connected to a direct current power source;
**characterized in that** the fluid is a gas and the device is equipped with a hydrogen peroxide nebulizer (7) fitted with a sonolysis head; and
**in that** the coating is applied to the blades (21) of the pump or compressor (2).

2. A fluid treatment device in accordance with claim 1, **characterised in that** the coating is made of titanium oxides, metal nitrides, strontium titanate or other metal oxides with the ability to absorb radiation in the visible spectrum.

3. A fluid treatment device in accordance with claim 1, **characterised in that** the coating is also comprised of a layer of titanium, cobalt, copper, silver, zinc or aluminium arranged applied between the titanium oxide or metal nitride on the support (8).

4. A fluid treatment device in accordance with claim 1, **characterised in that** the fluid outlet (3) is an air jet.

5. A fluid treatment device in accordance with claim 3, **characterised in that** the coating is applied by vapour deposition.

6. -A fluid treatment device in accordance with claim 1, **characterised in that** it is equipped with a filter (6).

7. -A fluid treatment device in accordance with claim 1, **characterised in that** the device is equipped with an ozonation unit (5).

8. A fluid treatment device in accordance with claim 7, **characterised in that** the nebulizer (7) is installed next to the ozonation unit (5).

9. A fluid treatment device in accordance with claim 8, **characterised in that** the nebulizer (7) and the ozonation unit (5) are placed upstream of the coating.

10. -A fluid treatment device in accordance with claim 3, **characterised in that** the coating is made of titanium dioxide and the underlying layer is made of titanium.

11. -A fluid treatment device in accordance with claim 1, **characterised in that** the coating is connected to a direct current power source and furthermore the device is equipped with a UV light (4) directed at the coating.

12. -A fluid treatment device in accordance with claim 1, **characterised in that** the sonolysis head is configured to generate a square wave with a frequency of 640KHz.

## Patentansprüche

1. Eine Fluidbehandlungsvorrichtung mit einem Fluideinlass (1), einer Pumpe oder einem Kompressor (2) und einem Fluidauslass (3), wobei die Vorrichtung einen Träger (8) mit einem Innenbereich mit einer Beschichtung aus Materialien mit Elektrophotokatalyse-Eigenschaften, modifiziert mit Übergangsmetalldimeren und -trimeren, umfasst, die Strahlung in der Ultraviolettfrequenz emittiert, wobei der Träger (8) entweder:
mit der Erde verbunden und durch UV-Licht (4) beleuchtet ist oder
mit einer Gleichstromleistungsquelle verbunden ist;
**dadurch gekennzeichnet, dass** das Fluid ein Gas ist und die Vorrichtung mit einem Wasserstoffperoxidzerstäuber (7) ausgestattet ist, der mit einem Sonolysekopf ausgestattet ist; und
dadurch, dass die Beschichtung auf die Schaufeln (21) der Pumpe oder des Kompressors (2) aufgebracht wird.

2. Eine Fluidbehandlungsvorrichtung nach Anspruch 1, **dadurch gekennzeichnet, dass** die Beschichtung aus Titanoxiden, Metallnitriden, Strontiumtitanat oder anderen Metalloxiden mit der Fähigkeit besteht, Strahlung im sichtbaren Spektrum zu absorbieren.

3. Eine Fluidbehandlungsvorrichtung nach Anspruch 1, **dadurch gekennzeichnet, dass** die Beschichtung ebenso aus einer Schicht aus Titan, Kobalt, Kupfer, Silber, Zink oder Aluminium besteht, die zwischen dem Titanoxid oder Metallnitrid auf dem Träger (8) aufgebracht ist.

4. Eine Fluidbehandlungsvorrichtung nach Anspruch 1, **dadurch gekennzeichnet, dass** der Fluidauslass (3) ein Luftstrahl ist.

5. Eine Fluidbehandlungsvorrichtung nach Anspruch 3, **dadurch gekennzeichnet, dass** die Beschichtung durch Gasphasenabscheidung aufgebracht wird.

6. Eine Fluidbehandlungsvorrichtung nach Anspruch 1, **dadurch gekennzeichnet, dass** sie mit einem Filter (6) ausgestattet ist.

7. Eine Fluidbehandlungsvorrichtung nach Anspruch 1, **dadurch gekennzeichnet, dass** die Vorrichtung mit einer Ozonisierungseinheit (5) ausgestattet ist.

8. Eine Fluidbehandlungsvorrichtung nach Anspruch 7, **dadurch gekennzeichnet, dass** der Zerstäuber (7) neben der Ozonisierungseinheit (5) installiert ist.

9. Eine Fluidbehandlungsvorrichtung nach Anspruch 8, **dadurch gekennzeichnet, dass** der Zerstäuber (7) und die Ozonisierungseinheit (5) der Beschichtung vorgeschaltet sind.

10. Eine Fluidbehandlungsvorrichtung nach Anspruch 3, **dadurch gekennzeichnet, dass** die Beschichtung aus Titandioxid und die darunter liegende Schicht aus Titan besteht.

11. Eine Fluidbehandlungsvorrichtung nach Anspruch 1, **dadurch gekennzeichnet, dass** die Beschichtung mit einer Gleichstromleistungsquelle verbunden ist und die Vorrichtung ferner mit einem auf die Beschichtung gerichteten UV-Licht (4) ausgestattet ist.

12. Eine Fluidbehandlungsvorrichtung nach Anspruch 1, **dadurch gekennzeichnet, dass** der Sonolysekopf so konfiguriert ist, dass er eine Rechteckwelle mit einer Frequenz von 640 KHz erzeugt.

## Revendications

1. Un dispositif de traitement de fluide avec une admission de fluide (1), une pompe ou un compresseur (2) et une sortie de fluide (3), le dispositif comprenant un support (8) avec une zone interne avec un revêtement de matériaux ayant des propriétés d'électrophotocatalyse, modifiés avec des dimères et des trimères de métaux de transition, qui émet du rayonnement dans la fréquence ultraviolette, le support (8) étant soit :
connecté à la terre et éclairé par une lumière UV (4) ou
connecté à une source d'alimentation en courant continu ;
**caractérisé en ce que** le fluide est un gaz et le dispositif est équipé d'un nébuliseur de peroxyde d'hydrogène (7) équipé d'une tête de sonolyse ; et
**en ce que** le revêtement est appliqué sur les aubes (21) de la pompe ou du compresseur (2).

2. Un dispositif de traitement de fluide selon la revendication 1, **caractérisé en ce que** le revêtement est constitué d'oxydes de titane, de nitrures de métal, de titanate de strontium ou d'autres oxydes de métal ayant la capacité d'absorber du rayonnement dans le spectre visible.

3. Un dispositif de traitement de fluide selon la revendication 1, **caractérisé en ce que** le revêtement est également composé d'une couche de titane, de cobalt, de cuivre, d'argent, de zinc ou d'aluminium disposée entre l'oxyde de titane ou le nitrure de métal sur le support (8).

4. Un dispositif de traitement de fluide selon la revendication 1, **caractérisé en ce que** la sortie de fluide (3) est un jet d'air.

5. Un dispositif de traitement de fluide selon la revendication 3, **caractérisé en ce que** le revêtement est appliqué par dépôt en phase vapeur.

6. Un dispositif de traitement de fluide selon la revendication 1, **caractérisé en ce qu'**il est équipé d'un filtre (6).

7. Un dispositif de traitement de fluide selon la revendication 1, **caractérisé en ce que** le dispositif est équipé d'une unité d'ozonation (5).

8. Un dispositif de traitement de fluide selon la revendication 7, **caractérisé en ce que** le nébuliseur (7) est installé à côté de l'unité d'ozonation (5).

9. Un dispositif de traitement de fluide selon la revendication 8, **caractérisé en ce que** le nébuliseur (7) et l'unité d'ozonation (5) sont placés en amont du revêtement.

10. Un dispositif de traitement de fluide selon la revendication 3, **caractérisé en ce que** le revêtement est réalisé en dioxyde de titane et la couche sous-jacente est réalisée en titane.

11. Un dispositif de traitement de fluide selon la revendication 1, **caractérisé en ce que** le revêtement est connecté à une source d'alimentation en courant continu et en outre le dispositif est équipé d'une lumière UV (4) dirigée vers le revêtement.

12. Un dispositif de traitement de fluide selon la revendication 1, **caractérisé en ce que** la tête de sonolyse est configurée pour générer une onde carrée avec une fréquence de 640KHz.
